# EUROPÄISCHE PATENTSCHRIFT

(11) **EP 1 139 780 B1**
(45) Veröffentlichungstag und Bekanntmachung des Hinweises auf die Patenterteilung: **13.09.2006**
(21) Anmeldenummer: 99969217.1
(22) Anmeldetag: 18.12.1999
(51) Int. Cl.: A23K 1/00, A23K 1/165

(54) **VERFAHREN ZUR HERSTELLUNG VON ENZYMHALTIGEN GRANULATEN**
METHOD FOR PRODUCING GRANULATES CONTAINING ENZYMES
PROCEDE DE PRODUCTION DE GRANULES CONTENANT DES ENZYMES

(30) Priorität: 22.12.1998 DE 19859385
(43) Veröffentlichungstag der Anmeldung: 10.10.2001
(73) Patentinhaber: BASF Aktiengesellschaft, 67056 Ludwigshafen (DE)
(72) Erfinder: HARZ, Hans-Peter, D-67373 Dudenhofen (DE); HEINZL, Wolfgang, D-67157 Wachenheim (DE); SCHÖNER, Franz-Josef, D-67480 Edenkoben (DE); BETZ, Roland, D-67150 Niederkirchen (DE); KESSLER, Thomas, D-67105 Schifferstadt (DE)
(86) Internationale Anmeldenummer: PCT/EP1999/010139
(87) Internationale Veröffentlichungsnummer: WO 2000/036927

(56) Entgegenhaltungen:
- WO-A-98/54980
- PATENT ABSTRACTS OF JAPAN vol. 012, no. 186 (C-500), 31. Mai 1988 (1988-05-31) & JP 62 294039 A (TOSHIBA MACH CO LTD;OTHERS: 01), 21. Dezember 1987 (1987-12-21)

## Beschreibung

Die vorliegende Erfindung betrifft ein neues Verfahren zur Herstellung von für die Tierernährung geeigneten enzymhaltigen Granulaten durch Vermischen mindestens eines Enzyms mit einem Trägermaterial und Extrusion dieser Mischung.

Die Verwendung von enzymhaltigen Materialien in der Tierernährung ist allgemein bekannt. Solche Mittel wirken verdauungsfördernd und verbessern dadurch die Futterverwertung und die Energieaufnahme aus dem Futter.

In der EP-B 257 996 sind enzymhaltige Vormischungen für die Tierernährung beschrieben, die durch Absorption von wäßrigen Enzymlösungen auf einen Träger auf Getreidebasis mit anschließender Pelletierung des Träger-Enzym-Komplexes erhalten werden.

Aus der WO 97/12958 ist die Herstellung von enzymhaltigen Mikrogranulaten durch Wirbelbettgranulierung bekannt, wobei eine Vormischung aus einer wäßrigen Enzymlösung und einem Bindemittel im Wirbelbett auf einen Träger aufgebracht wird und das so erhaltene Material anschließend mit einem Überzug aus einem wasserlöslichen Polymer versehen wird.

In der EP-B 564 476 ist ein Verfahren zur Herstellung von Enzymgranulaten durch Extrudieren eines durch Vermischen einer Fermentationsbrühe mit Zuschlagstoffen entstandenen rieselfähigen Enzymvorgemischs beschrieben.

Die bekannten Verfahren sind jedoch relativ aufwendig und die entstehenden Produkte lassen hinsichtlich der Produktstabilität und der Korngrößenverteilung noch Raum für Verbesserungen.

Auch WO9854980 offenbart die Herstellung enzymhaltiger Granulate für die Tierernährung. Aufgabe der vorliegenden Erfindung war es ein Verfahren zur Herstellung von enzymhaltigen Granulaten für den Einsatz in der Tierernährung, welches zu verbesserten Produkteigenschaften führt, zu finden.

Demgemäß wurde ein Verfahren zur Herstellung von für die Tierernährung geeigneten Enzymgranulaten durch Vermischen mindestens eines Enzyms mit einem Trägermaterial und Extrusion dieser Mischung gefunden, welches dadurch gekennzeichnet ist, daß man zunächst das Trägermaterial in einem mit mindestens einer horizontal angeordneten Schnecke ausgerüsteten Schneckenkneter plastifiziert, dann das Enzym in den Schneckenkneter einbringt und mit dem plastifizierten Trägermaterial zu einer homogenen Masse verarbeitet und diese extrudiert.

Das erfindungsgemäße Verfahren eignet sich prinzipiell zur Verarbeitung aller Enzyme oder Enzymmischungen, insbesondere für solche,die für die Tierernährung geeignet sind. Als Enzyme kommen beispielsweise in Betracht:

Oxidoreduktasen, Transferasen, Lyasen, Isomerasen oder Ligasen, und insbesondere Hydrolasen. Hydrolasen, also Enzyme, die eine hydrolytische Spaltung von Bindungen verursachen können, sind beispielsweise Esterasen, Glykosidasen, Etherhydrolasen, Proteasen, Amidasen, Aminidasen, Nitrilasen oder Phosphatasen. Zu den Glyksidasen gehören beispielsweise sowohl endo- als auch exo-Glucosidasen, die sowohl α- als auch β-glykosidische Bindungen spalten können, z.B. Amylase, Maltase, Cellulase, Endo-Xylanase, β-Glucanase, Mannanase, oder Lysozym, weiterhin Galactosidase oder β-Glucuronidasen. Bevorzugt werden Nichtstärkepolysaccharid-spaltende Enzyme verarbeitet. Ganz besonders bevorzugt ist Phytase.

Die Enzyme werden im allgemeinen als wäßrige Lösungen eingesetzt, vorzugsweise als wäßrige Retentate einer Ultrafiltration, wie sie auf an sich bekannte Weise aus Fermentationsprozessen erhalten werden.

Die Trockenmasse an Enzym liegt im Bereich von 15 bis 35, bevorzugt 20 bis 25 Gew.-%, bei 10.000 bis 35.000 IU/g. die wäßrigen Enzymlösungen werden in solchen Mengen eingesetzt, daß im Granulat 1000 bis 8000 Einheiten/g enthalten sind.

Man kann die Enzyme aber auch in den entsprechenden Mengen in Form von Trockenpulvern einsetzen.

Besonders bevorzugt werden erfindungsgemäß Phytase-Ultrafiltrate.

Als Hilfsstoffe für die Trägermatrix eignen sich prinzipiell alle für die Tierernährung geeigneten Hilfsstoffe, die unter den pH-Bedingungen im Verdauungstrakt der Tiere die Enzyme ausreichend schnell freisetzen und eine gute Verträglichkeit mit den Enzymen aufweisen. Geeignete Hilfsstoffe sind beispielsweise polymere Bindemittel wie Polyvinylpyrrolidon, Copolymere aus N-Vinylpyrrolidon und N-Vinylacetat wie beispielsweise ein VP-VAc-Copolymer 6+4, oder Cellulosederivate wie Hydroxypropylcellulose oder bevorzugt Hydroxypropylmethylcellulose, weiterhin Polyvinylacetat, Polyvinylalkohol, Polyacrylate oder Polymethacrylate.

Ebenso eignen sich Getreideprodukte wie beispielsweise Weizengriesskleie, oder Milchprodukte wie z.B. Magermilchpulver. Als Trägermaterialien eigene sich insbesondere auch Fette und Wachse.

Ebenso eignen sich alle Arten von Stärke, weiterhin Oligosaccharide wie die Dextrine sowie Di- und Monosaccharide wie z.B. Saccharose, Lactose, Fructose, Galactose, Mannose oder Sorbose. Als Materialien für die Trägermatrix kommen weiterhin niedermolekulare Polyethylenglykole mit Molekulargewichten von 200 bis 20000 g/mol, bevorzugt 5000 bis 8000 g/mol in Betracht. Ebenso eigenen sich Polyoxyethylen-polyoxypropylen-Blockcopolymere, die auch als Poloxamere bekannt sind.

Besonders bevorzugt werden als Trägermaterialien Mischungen aus Stärke und Polyethylenglykolen eingesetzt.

Man kann den Trägermaterialien weiterhin auch Gelatine oder andere Proteine in Mengen von bis zu 50 Gew.-% zusetzen. Ebenso können die Trägermaterialien anorganische Zusatzstoffe wie Kalk, Bentonite oder Silicate enthalten.

Der Anteil der Trägermaterialien am Gesamtgewicht der Granulate beträgt, bezogen auf das Trockengewicht der Granulate, bis zu 99,9 Gew.-%, bevorzugt 50 bis 99 Gew.-%, besonders bevorzugt 88 bis 98 Gew.-%.

Zusätzlich können den Trägermaterialien noch Mineralstoffe wie beispielsweise Magnesiumsulfat, Zinksulfat oder Natriumsulfat zugegeben werden. Weiterhin kann zur Einstellung oder pH-Werte auch die Zugabe von weiteren Salzen wie beispielsweise Acetaten, Tartraten oder Citraten hilfreich sein.

Erfindungsgemäß kann man alle Komponenten für das Trägermaterial zu einer Vormischung zusammengeben und in den Schneckenkneter einbringen oder auch einzelne Hilfsstoffkomponenten zu einem späteren Zeitpunkt während des Extrudierens hinzufügen.

Zum Vermischen und Homogenisieren der Trägermaterialien und des enzymhaltigen Ultrafiltrats oder des Enzymtrockenpulvers eignen sich alle Schneckenkneter mit mindestens einer horizontal angebrachten Schnecke. Das erfindungsgemäße Verfahren erlaubt neben dem Einsatz von Einschnecken-Extrudern auch den Einsatz von kämmenden oder nichtkämmenden Mehrwellenextrudern, insbesondere von Zweischneckenextrudern die gleichsinnig oder gegensinnig drehend sein können.

Erfindungsgemäß werden zunächst die Komponenten der Trägermatrix in einem Schneckenkneter durch Zuführung von mechanischer und thermischer Energie plastifiziert. Je nach Art der Trägermaterialien kann dies beispielsweise durch Schmelzen der Komponenten erfolgen. Die Plastifizierung kann bei Temperaturen im Bereich von 20 bis 250°C durchgeführt werden.

Nach dem die Trägermaterialien bzw. deren Gemische ausreichend plastifiziert und homogenisiert sind, erfolgt eine Abkühlung der Masse und anschließend der Eintrag des Ultrafiltrats oder des Enzymtrockenpulvers in den Schneckenkneter. Der Eintrag kann beispielsweise so erfolgen, daß das wässrige Ultrafiltrat oder das Enzymtrockenpulver über geeignete Dosiervorrichtungen dem Schneckenkanal zugeführt wird. Die Massetemperatur der Trägermaterialien beträgt an dieser Stelle bevorzugt nicht über 70°C, besonders bevorzugt 20 bis 60°C.

Die Länge des Schneckenkanals wird vorzugsweise so gewählt, daß die Gesamtverweilzeit der Masse im Extruder kleiner 10 min., bevorzugt kleiner 2 min. beträgt. Insbesondere in Extruderschüssen, in denen die Massetemperatur größer 30°C beträgt, soll die Verweilzeit kleiner 2 min. betragen.

Im Anschluß an die Homogenisierung erfolgt in der Regel der Austrag aus dem Extruder und die Formgebung. Dabei kann die homogenisierte Masse durch eine Düse oder durch eine Lochplatte extrudiert werden. Die austretenden Stränge können durch Heiß- oder Kaltabschlag zu gleichteiligen Granulaten verformt werden.

Man kann auch durch Anlegen eines Vakuums eine Teiltrocknung der Masse im Extruder vornehmen. Man kann auch direkt Granulate aus dem Extruder austragen, und zwar dergestalt, daß die Schnecke an ihrem zum Extruderkopf hingelegenen Ende mit Mahlelementen ausgerüstet ist. Die Extrusion erfolgt dann über den offenen Extruderkopf.

Gewünschtenfalls können die so erhaltenen Granulate anschließend einem zusätzlichen Trocknungsprozess unterworfen werden.

Die nach dem erfindungsgemäßen Verfahren erhaltenen enzymhaltigen Granulate weisen mittlere Korngrößen im Bereich von 500 bis 2000 µm, vorzugsweise von 500 bis 1000 µm auf.

Sie eignen sich zum Einsatz in Futtermitteln für Geflügel, Schweine, Kälber oder zum Einsatz in der Aquakultur, beispielsweise in Forellen- oder Lachsfutter.

Zur Herstellung der Futtermittelpellets werden die Enzymgranulate mit einem Futtermittel vermischt. Im allgemeinen werden z.B. bei Phytase enthaltenden Futtermitteln Gehalte von einigen zehn bis einigen hundert ppm Phytase eingestellt. Anschließend wird das Futter pelletiert, wobei alle handelsüblichen Typen von Pelletpressen zum Einsatz kommen können. All diesen Pelletpressen ist gemeinsam, daß zunächst das Futter durch Dampfeinleitung konditioniert und anschließend durch die Matrize gepresst wird. Je nach Matrize können so Pellets von 2 bis 12 mm Korngröße hergestellt werden. Beim Pressen der Pellets durch die Matrize wird die maximale Temperaturbelastung des Pelletierprozesses erreicht. Hierbei können Temperaturen von 60 bis 100°C erzielt werden.

Zur Beurteilung der Stabilität der Futtermittel-Enzyme beim Pelletieren wurde eine Standardpelletierung festgelegt. Dabei wird zur Verbesserung der analytischen Gehaltsbestimmung die Enzymdosierung im Futter erhöht. Der Pelletierprozess wird so durchgeführt, daß stets eine Pellettemperatur von 80°C erreicht wird. Vom pelletierten Futter wird die Aktivität des Enzyms im Vergleich zur Ausgangsaktivität bestimmt, wobei gegebenenfalls um den Gehalt an nativem Enzym korrigiert wird. Zur Kontrolle wird stets ein Standard mitpelletiert und analysiert.

Aufgrund ihrer günstigen Korngrößenverteilung und der Einbettung des Enzymmaterials in eine stabilisierende Matrix weisen die Granulate eine gute Thermostabilität, insbesondere bei der Verarbeitung zu Futtermittelpellets, auf.

### Beispiele

### Allgemeine Verfahrensbeschreibung

Die Herstellung der Granulate erfolgt in einem Doppelschneckenextruder des Typs ZSK 30, Fa. Werner & Pfleiderer. Der Extruder wird mit dem folgenden Temperaturprofil betrieben:
Schuß 1: 40°C; Schuß 2: 100°C; Schuß 3: 120°C; Schuß 4: 60°C;
Schuß 5: 45°C; Schuß 6: 45°C; Schuß 7: 45°C

Die Zugabe der Enzymlösung erfolgt in Schuß 6. Die Masse wird durch eine Lochplatte extrudiert und getrocknet.

Auf diese Weise lassen sich Formulierungen der folgenden Zusammensetzung verarbeiten:

**Formulierung 1**

| | |
|---|---|
| Phytase | 4 Gew.-% |
| Maisstärke | 76 Gew.-% |
| Polyethylenglykol 6000 | 20 Gew.-% |

**Formulierung 2**

| | |
|---|---|
| Phytase | 5 Gew.-% |
| Maisstärke | 88,6 Gew.-% |
| Lutrol® F127) | |
| (Poloxamer 407) | 2,4 Gew.-% |
| MgSO₄ | 4 Gew.-% |

**Formulierung 3**

| | |
|---|---|
| Phytase | 6 Gew.-% |
| Maisstärke | 70 Gew.-% |
| Lutrol® F127) | |
| (Poloxamer 407) | 20 Gew.-% |
| MgSO₄ | 4 Gew.-% |

**Formulierung 4**

| | |
|---|---|
| Phytase | 6 Gew.-% |
| Maisstärke | 76 Gew.-% |
| Hydroxypropylmethylcellulose | 15 Gew.-% |
| MgSO₄ | 3 Gew.-% |

**Formulierung 5**

| | |
|---|---|
| Phytase | 5 Gew.-% |
| Maisstärke | 90 Gew.-% |
| Fett | 5 Gew.-% |

**Formulierung 6**

| | |
|---|---|
| Phytase | 7 Gew.-% |
| Maisstärke | 68 Gew.-% |
| Lutrol® F68 | |
| (Poloxamer 188) | 25 Gew.-% |

**Formulierung 7**

| | |
|---|---|
| Phytase | 8 Gew.-% |
| Maisstärke | 75 Gew.-% |
| Hydroxypropylmethylcellulose | 7,5 Gew.-% |
| Copovidone | 7,5 Gew.-% |
| MgSO₄ | 2 Gew.-% |

**Formulierung 8**

| | |
|---|---|
| Phytase | 10 Gew.-% |
| Maistärke | 83 Gew.-% |
| Hydroxypropymethylcellulose | 2,5 Gew.-% |
| Copovidone | 2,5 Gew.-% |
| MgSO4 | 2 Gew.-% |

**Formulierung 9**

| | |
|---|---|
| Phytase | 6,5 Gew.-% |
| Maisstärke | 90 Gew.-% |
| Polyvinylalkohol | 3,5 Gew.-% |

### Pelletierversuch

Die Granulate werden zusammen mit einem Futtermittel (Zusammensetzung siehe nachstehende Tabelle)in einer Pelletiermaschine der Firma CPM (California Pellet Mill Company) bei Temperaturen im Bereich von 70 bis 80°C zu Pellets verarbeitet. Die Bestimmung der Enzymretention (jeweils korrigiert um native Phytase) erfolgt wie in "Bestimmung der Phytaseaktivität in Futtermitteln und Vormischungen, VDLUFA-Methodenbuch, Band III, 4. Erg., 1997" beschrieben.

**Tabelle: Zusammensetzung Schweinemastfutter**

| Komponenten | Gew.-% |
|---|---|
| Mais | 20,70 |
| Gerste | 40,00 |
| Tapioka | 10,00 |
| Hafer | 10,00 |
| Sojaschrot | 13,00 |
| Fischmehl | 3,00 |
| Weizenkleie | 0,84 |
| Sojaöl | 0,50 |
| Kohlensaurer Futterkalk | 1,20 |
| Viehsalz | 0,20 |
| Spurenelemente | 0,06 |
| DL-Methionin | 0,05 |
| Cholinchlorid (50 %) | 0,05 |
| Propionsäure | 0,40 |

## Patentansprüche

1. Verfahren zur Herstellung von für die Tierernährung geeigneten enzymhaltigen Granulaten durch Vermischen mindestens eines Enzyms mit einem Trägermaterial und Extrusion dieser Mischung, **dadurch gekennzeichnet, daß** man zunächst das Trägermaterial in einem mit mindestens einer horizontal angeordneten Schnecke ausgerüsteten Schneckenkneter plastifiziert, dann das Enzym in den Schneckenkneter einbringt und mit dem plastifizierten Trägermaterial zu einer homogenen Masse verarbeitet und diese extrudiert.

2. Verfahren nach Anspruch 1, **dadurch gekennzeichnet, daß** das Enzym in Form einer wäßrigen Lösung zugegeben wird.

3. Verfahren nach einem der Ansprüche 1 oder 2, **dadurch gekennzeichnet, daß** man als Trägermaterial eine Mischung aus Stärke und mindestens einem weiteren thermoplastisch verarbeitbaren Polymer einsetzt.

4. Verfahren nach Anspruch 3, **dadurch gekennzeichnet, daß** man Mischungen aus Stärke und Polyethylenglykol einsetzt.

5. Verfahren nach Anspruch 3, **dadurch gekennzeichnet, daß** man Mischungen aus Stärke und Hydroxypropylmethylcellulose einsetzt.

6. Verfahren nach einem der Ansprüche 1 bis 5, **dadurch gekennzeichnet, daß** man vor Zugabe des Enzyms die Temperatur des plastifizierten Trägermaterials auf maximal 70°C absenkt.

7. Verfahren nach einem der Ansprüche 1 bis 6, **dadurch gekennzeichnet, daß** die Gesamtverweilzeit im Schneckenkanal des Extruders <10 min beträgt.

8. Verfahren nach einem der Ansprüche 1 bis 7, **dadurch gekennzeichnet, daß** als Enzym Phytase eingesetzt wird.

## Claims

1. A process for producing enzyme-containing granules suitable for animal nutrition, by mixing at least one enzyme with a carrier material and extruding this mixture, which comprises first plasticizing the carrier material in a screw extruder equipped with at least one horizontal screw, then introducing the enzyme into the screw extruder and processing it together with the plasticized carrier material to form a homogeneous mixture and extruding this.

2. The process according to claim 1, wherein the enzyme is added in the form of an aqueous solution.

3. The process according to either claim 1 or 2, wherein the carrier material is a mixture of starch and at least one further thermoplastic polymer.

4. The process according to claim 3, wherein mixtures of starch and polyethylene glycol are used.

5. The process according to claim 3, wherein mixtures of starch and hydroxypropyl methyl cellulose are used.

6. The process according to one of claims 1 to 5, wherein, before adding the enzyme, the temperature of the plasticized carrier material is reduced to at most 70°C.

7. The process according to one of claims 1 to 6, wherein the total residence time in the extruder screw barrel is <10 min.

8. The process according to one of claims 1 to 7, wherein the enzyme is phytase.

## Revendications

1. Procédé de préparation de granules contenant de l'enzyme appropriés pour l'alimentation des animaux, par mélange d'au moins une enzyme avec une matière de support et extrusion de ce mélange, **caractérisé en ce qu'**on plastifie tout d'abord la matière de support dans une pétrisseuse équipée d'au moins une vis agencée horizontalement, puis on introduit l'enzyme dans la pétrisseuse à vis et on la traite avec la matière de support plastifiée pour former une masse homogène et on extrude celle-ci.

2. Procédé suivant la revendication 1, **caractérisé en ce que** l'enzyme est ajoutée sous la forme d'une solution aqueuse.

3. Procédé suivant l'une des revendications 1 ou 2, **caractérisé en ce que**, comme matière de support, on met en oeuvre un mélange d'amidon et d'au moins un autre polymère qui peut être traité de manière thermoplastique.

4. Procédé suivant la revendication 3, **caractérisé en ce qu'**on met en oeuvre des mélanges d'amidon et de polyéthylèneglycol.

5. Procédé suivant la revendication 3, **caractérisé en ce qu'**on met en oeuvre des mélanges d'amidon et d'hydroxypropylméthylcellulose.

6. Procédé suivant l'une des revendications 1 à 5, **caractérisé en ce que**, avant l'addition de l'enzyme, on diminue la température de la matière de support plastifiée à au maximum 70°C.

7. Procédé suivant l'une des revendications 1 à 6, **caractérisé en ce que** le temps de séjour total dans le canal à vis de l'extrudeuse est <10 minutes.

8. Procédé suivant l'une des revendications 1 à 7, **caractérisé en ce qu'**on met en oeuvre de la phytase, comme enzyme.
